# EUROPEAN PATENT SPECIFICATION

(11) **EP 1 689 759 B1**
(45) Date of publication and mention of the grant of the patent: **13.07.2016**
(21) Application number: 03777052.6
(22) Date of filing: 04.12.2003
(51) Int. Cl.: C07F 9/38, A61K 31/663, A61P 19/00, C07F 9/6506

(54) **METHOD FOR PRODUCING PURE DISODIUM PAMIDRONATE**
VERFAHREN ZUR HERSTELLUNG VON REINEM DINATRIUM PAMIDRONAT
PROCEDES DE PRODUCTION DE PAMIDRONATE DE DISODIUM PUR

(43) Date of publication of application: 16.08.2006
(73) Proprietor: Mustafa Nevzat Ilac Sanayii A.S., 80290 Gayrettepe (TR)
(72) Inventor: ÜNLÜSAYIN, Irfan, Da:1, Acibadem, Kadikoy, Istanbul (TR)
(74) Representative: Kodron, Felix
(86) International application number: PCT/IB2003/005803
(87) International publication number: WO 2005/054260

(56) References cited:
- EP-A- 1 236 733
- WO-A-00/34293
- WO-A-01/42134
- WO-A-03/050127
- WO-A-03/053985
- US-A- 4 639 338
- US-A1- 2003 138 462

## Description

Medical applications of biphosphonates as described in the literature cover a wide range of indications such as hypercalcemia of malignancy, bone marrow edema syndrome, osteolytic bone lesions of multiple myeloma, osteolytic bone metastases of breast cancer, Paget's disease of bone all of which to be treated by injection. Hypercalcemia of malignancy, osteoporosis, Paget's disease of bone can also be treated by appropriate oral dosage forms of disodium pamidronate.
Disodium pamidronate is effective in moderate or severe hypercalcemia of malignancy and in treating bone pain associated with various tumors. Preliminary studies also suggest that anti-TNF therapy may hold great promise in severe ankylosant spondilitis unresponsive to conventional therapy.

Thus, along with such progress in medical therapy of cancer and other diseases, biphosponates are becoming more and more important, see e.g. patent application WO 00/71104 A2 (PCT/EP00/04562) of OKUNO TETSUI & al.. They are employing and claiming such compounds for the treatment of angiogenesis in patients in need of such treatment, e.g. a tumor patient or a patient suffering from inflammatory disease, which comprises administering, preferably via an intra-arterial route, an effective amount of a biphosphonate, e.g. pamidronic acid or zoledronic acid or salts or hydrates thereof, to the patient. In their patent application they claim more than 12 biphosphonates, however, they do not specify them in terms of purity and stability.

Neither are such specifications given by H. JOMAA (DE 198 28 450 A1, priority data 26.06.98), claiming numerous biphosphonic acids and their derivates for treatment of 21 different auto-immuno-diseases and allergies.

However, stable and pure ingredients are prerequisites for the manufacture of galenic formulations. From the patent literature (others than OKUNO & al., above, where the aspects of synthesis and purities of biphosphonates are not disclosed) one may get the impression that making stable sodium salts from pamidronic acid involves multistep sometimes harsh procedures which may not minimize impurities in the final product.

For example, in 2003 KIM & al. received an US patent 6,534,674 (also published as WO 01/42134 A) where they reflux pamidronic acid in absolute methanol containing sodium methoxide for 12 hours, then they cool down to 0°C, filter and dry in the oven at 60°C. The product thus obtained they use for making a 3-4 hydrate, as follows: They add water and heat the mixture up to 95°C. Insoluble material is filtered off and while the filtrate is cooling gradually to normal temperature, a crystallized suspension is obtained. Then after several more steps crystalline disodium pamidronate in form of its "stable" 3-4 hydrate is obtained.

If such a multistep procedure along with the dangerous aspect of employing sodium alcoholate and organic solvents at boiling temperature can be considered an easy procedure for just a neutralization reaction remains to be answered, although KIM & al. emphasize that with such method, pH adjustment is not required, as the reaction is proceeding in a quantitative way as to the formation of the disodium salt of pamidronic acid. Nevertheless, they present an alternative method to produce really disodium salt, namely by neutralizing pamidronic acid with sodium hydroxide unto the pH range of 7.5 about 8.5, preferably 8.1 about 8.3, which neutralization reaction they carry out at a temperature of 90°C and the mixture was adjusted to pH 7.5 about 8.5.

According to the fact that pH is dependant on temperature, and from the data given in the above patent, it is not really evident to the reader if this dependency has been taken into consideration by the inventors (e.g. pH 8.2 at 25°C would be pH 7.55 at 95°C; and pH 8.2 at 95°C would be pH 8.8 at 25°C, which is of course very much dependent on the nature of the respective acid and its salts, in this case the disodium pamidronate).

Special emphasis KIM & al. put on the stability aspect, claiming that the 3-4 hydrate is much more stable than the known disodium pentahydrate, as the 3-4 hydrate shows an increased validity time in storage or when used as an ingredient in pharmaceutical formulation; and it can be conveniently formulated as oral preparation or as soft capsule in addition to injection formulation.

In 2002, CARACCIA & al. applied for a European Patent (EP 1 236 733 A1) claiming a process for preparation of a stable disodium pamidronate comprising the following steps:
Treatment of an aqueous suspension of pamidronic acid with NaOH aqueous solution until obtaining a clear solution.

Filtration of the resulting solution, addition of a solvent (e.g. 4 volumes of n-octanol), and subsequent azeotropic distillation, distilling off most of the water/octanol.
Cooling of the resulting solution and precipitation of the product, which contains 5-12 % water, denominated as a dihydrate, which is claimed to be stable.

It is not very likely that this procedure all the octanol added would have been removed in the product. More likely there will be traces of octanol in CARACCIA'S dihydrate.

As far as KIM'S "stable" 3-4 hydrate and CARACCIA'S "stable" dihydrate disodium salts of pamidronic acid are concerned, they may be in contradiction with the findings of STAHL & al. (US Patent 4,639,338 in 1987) who made some systematic study in their pursuit of a stable crystalline disodium pamidronate.

STAHL & al. found an unstable dihydrate (modification B in their above patent) and an unstable 3-4 hydrate (modification C in their above patent).

Instability they defined in terms of a hygroscopic behavior (which is generally undesired a quality for the production of pharmaceutical formulations).

STAHL & al. (US Patent 4,639,338) are claiming a stable crystalline pentahydrate (modification E of their systematic study). This product became a drug substance marketed by Novartis under the trade name of AREDIA®. This pentahydrate is produced by a special way of crystallization in which the crystal formation has to be initiated at least at 50°C, and drying is carried out at normal or slightly elevated temperature.

It is this pentahydrate which is challenged by KIM'S & al. 3-4 hydrate, as shown above, in terms of its stability. It is this pentahydrate, which in form of AREDIA® has been used by OKUNO & al., in their example 5 of their patent application, with breast cancer patients, tongue cancer patient and a case with right knee joint osteoarthrytis, as well.

Compared with CARACCIA'S or KIM'S method, the procedure of STAHL appears almost simple notwithstanding its rather sophisticated way of running temperatures along the crystallization process.

Vis-à-vis all these controversies in making stable crystals of pamidronate, it is not a surprise that there still is a hunt for feasible alternative methods focusing on anhydrous disodium pamidronate and their aqueous solutions (e.g. SHINAL in US patent 6,268,524 and US patent 6160165 A, which is also published as WO 00/34293 or GRASSI in WO 03/053985 A1 as well as ANZAGHI in WO 03/050127 A1), or focusing on more or less neutralized aqueous solutions of pamidronic acid.

Such developments focusing on intravenous infusion or injection and appropriate containers and ampoules for this purpose are being claimed by HANDRECK & al. (US Patent application 20030138462, filed 2002). In a similar fashion, MIREJOVSKY & al. (WO 02/087592; PCT/US02/13801 and US Patent Application 203/0069211 A1) applied for a patent in 2001 for their invention which includes a packaged liquid composition of disodium pamidronate in a sealed storage vessel having a special internal surface, to protect against elution of Ca²⁺ or Mg²⁺.

With HANDRECK'S and MIREJOVSKY'S methods, the crystallization step of disodium pamidronate is omitted and by omitting this step an essential step of purification is lost. It has to be kept in mind that pamidronic acid as it comes from the synthesis is not a pure substance and there are surprisingly little data on the purity of pamidronic acid and correspondingly there are little data on the purity of disodium pamidronate as well.

With respect to pamidronic acid (3-amino-1-hydroxypropane-1,1-diphosphonic acid), HAM & al. state that the method disclosed in DE Patent No. 2,130,794 gives a final product of very low purity, with an unidentified yellowish-red amorphous phosphorus-oxygen compound as a by-product (US Patent 5,792,885), a problem they hope to overcome with their method shown in the above patent in 1998.

To summarize, the state of the art of technology for producing salts of pamidronic acid is painstaking and the purity of the products is unsatisfactory and their stability is controversally discussed in the literature. So there is a need for a method, which avoids steps such as azeotropic distillation or reaction with sodium methoxide, and minimizes impurities and can be easily carried out in large-scale procedure.

This problem can be solved by the following invention that relates to a method for producing a pure, chemically stable form of disodium pamidronate.

We found that introducing an ion-exchange step is a significant progress versus the state of the art. Because it allows working at higher concentrations, at lower temperatures and under significant saving of time and energy and - most important - it avoids the addition of organic solvents at concentration and crystallization steps, and in addition to all these it removes the undesired divalent and trivalent metal ions.

The ion-exchange step has been introduced not for neutralization of the acid. The acid is treated with ammonia to obtain a highly concentrated solution of diammonium pamidronate due to its excellent solubility. By the ion exchange resin in its sodium form this diammonium pamidronate is converted into its disodium salt with almost no change of concentration. Thus crystallization of this disodium pamidronate can proceed without addition of alcohol, and/or concentration by evaporation.

The final product compares favorably against the British Pharmacopoeia standard. In order to prove the purity of our product we introduced additional methods for analysis of residual solvents, or determination of the divalent cation of calcium.

As already pointed out in the introduction, there are quite controversal findings (and claims) as to the stability of hydrates of disodium pamidronate, with STAHL & al. claiming a stable pentahydrate (and describing the dihydrate and the 3-4 hydrate as unstable); KIM & al. claiming a stable 3-4 hydrate and CARACCIA & al. claiming a stable dihydrate.

We feel that the reason for such a confusion may find its explanation in that maybe none of them had a stable hydrate because none of them had the disodium salt of pamidronic acid.

With our method of titrating the acid unto its equivalent point of diammonium salt and converting this into the disodium salt stoichiometrically by ion-exchange, we maybe the first of having realized the real disodium salt and its stable pentahydrate.

More particularly, the process of the invention comprises the following steps:
a) titration of the pamidronic acid with ammonia unto a pH of 7.50-7.60,
b) passing the resulting solution over the sodium form of an ion-exchange column at elevated temperature,
c) cooling down the resulting eluate containing disodium pamidronate to lower temperature until the disodium salt of pamidronic acid is crystallizing and
d) harvesting the crystals by centrifugation or vacuum filtration and drying in vacuum at 25°C.

The preferred concentration of ammonia used is such from 3 % to 30 %, preferably 12.5 %.

The total ammount of the ammonia used is such that the final pH of the neutralized solution is 7.55 ± 0.05 at 30°C.

The temperature at which the treatment of the acid with the ammonia is carried out is in a temperature range between 20°C to 40°C, preferably at 30°C.

The diammonium pamidronate solution is passed over the ion-exchange column at a temperature of 40°C to 60°C, preferably at 50°C.

The ion-exchange resin is a strongly acidic cation exchange resin in its sodium form.

The eluate containing disodium pamidronate is being crystallized by lowering the temperature to 5°C.

The crystals of disodium pamidronate are harvested and dried at room temperature under vacuum at 2 to 20 millibar.

The novel disodium pamidronate according to the present invention can be used for the preparation of medicaments for enteral and parenteral treatment of hypercalcemia of malignancy, bone marrow edema syndrome, osteolytic bone lesions of multiple myeloma, osteolytic bone metastases of breast cancer, Paget's disease of bone, osteoporosis, spondyloarthropathy including ankylosing spondilitis, vascular necrosis, McCune-Albright-Syndrome, bone pain associated with various tumors.

The preferred finished product forms of disodium pamidronate produced by the presented method are prepared as oral, liquid injectable and freeze dried injectable pharmaceutical dosage forms.

The present invention is illustrated in greater detail in the following examples: however, it is not intended to limit the scope of the present invention.

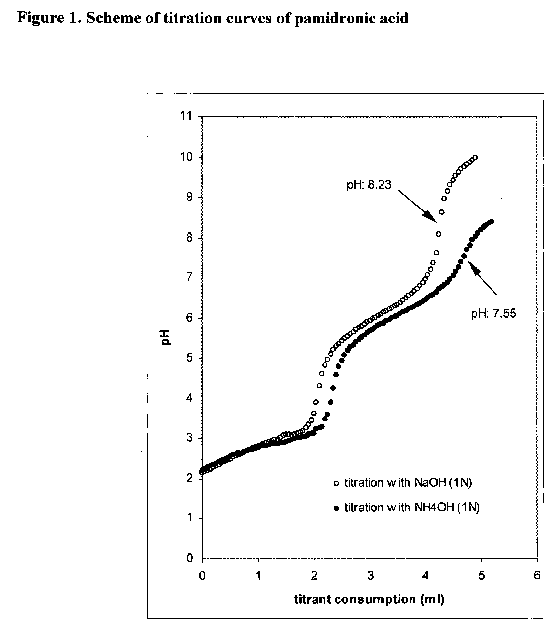

To make the disodium salt (and consequently its stable pentahydrate) neutralization process must be followed precisely with a calibrated pH Meter. From the titration curves was obtained the exact information on the pH of the aqueous solutions of the diammonium salt and the disodium salt of pamidronic acid. These points are pH 7.55 for the diammonium salt at 25°C and pH 8.23 for disodium salt at 25°C (see the arrows in the titration scheme, Figure 1).

### Detailed description of the invention:

We used pamidronic acid manufactured in our laboratory from phosphorus pentachloride, beta-alanine and phosphorous acid in the presence of chlorobenzene, according to the method of US 4,327,039, with some modifications of our own as to the crystallization conditions, which allowed us to obtain a product of high purity (>98 %) and a yield of 60-65 %.

This acid can directly be dissolved in 3.5 M ammonia at a molar ratio of acid to ammonia of 1:2; or titrated in water as slurry with ammonia at a concentration of 3 % to 30 %, preferably at a concentration of 12.5 % (w/v) up to pH range of 7.4 - 7.7, preferably 7.55 ± 0.05, preferably at room temperature (20°C - 25°C).

The titration step should be performed in preferably calcium free jacketed glass titration vessel. Due to the excellent solubility of diammonium salt of pamidronic acid, this allows to work at low temperature with highly concentrated solution. To work at a lower temperature may also reduce the solubility of impurities, which otherwise would lower the purity of the product.

When the neutralization step is completed, a highly concentrated diammonium solution is obtained and filtered under water-jet vacuum system through a PALL GH POLYPRO hydrophilic polypropylene membrane filter with the pore size of 0.45 micron for excluding insoluble reaction impurities and other materials. This clear filtrate is heated up to 50°C and passed through an ion-exchange resin column with a peristaltic pump at 50°C. This purification step provides a highly concentrated and purified disodium pamidronate at pH range of 8.1 to 8.5, mainly 8.3 (for details see Example 2).

This purified and concentrated disodium pamidronate solution is kept at a temperature of 50°C and fed to the crystallization system directly to obtain a pure crystalline and stable pentahydrate of disodium pamidronate at a very high yield (for details see Example 3).

The separated disodium pamidronate pentahydrate crystals are dried with vacuum filtration and further dried in the vacuum oven at a pressure range of 2 to 20 millibar at room temperature until the weight is constant.

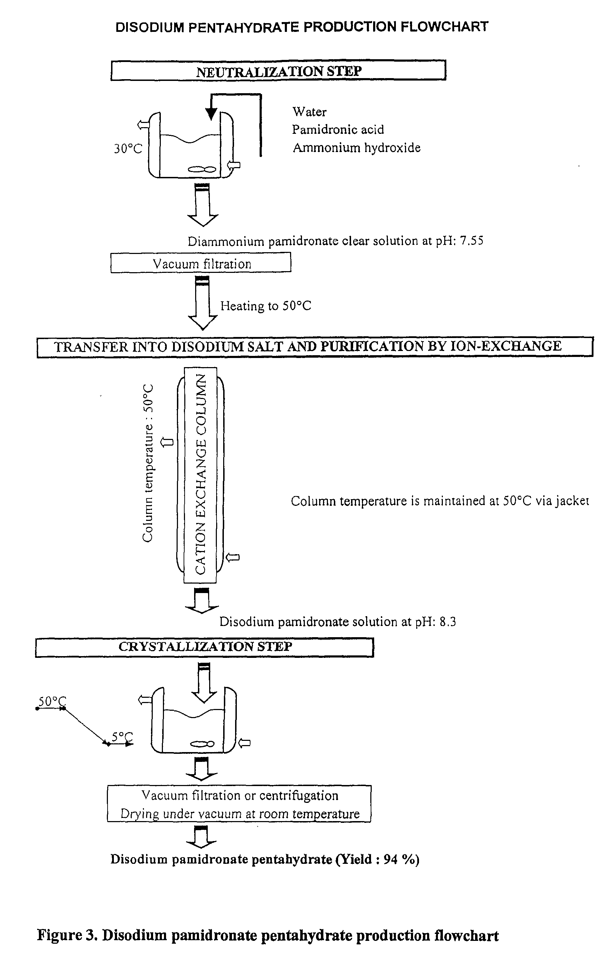

The overall process is given as flowchart in Figure 3.

The product, disodium pamidronate pentahydrate, C₃H₉NNa₂O₇P₂5H₂O, has a monograph in the British Pharmacopoeia, 2002, Vol. 1.

Three batches of disodium pamidronate pentahydrate were produced and analyzed for both assay and related substances with the methods given in the monograph.
None of the related substances (namely phosphate, phosphite and beta-alanine) were detected in any batch produced.

Water content as found by KF determination was in the range of 24.4 % to 24.6 %.

The absence of divalent cations was tested by complexometric titration with 0.01 N EDTA buffered at pH 10 with eriochrome-T as indicator as described in "Applied Complexometry" by R. Pribil (edited by R. Chalmers, Pergamon Press (1982)), with the result that the content of Ca²⁺ was below the detection limit of 40 ppm.

DSC data show only one endothermic peak between the range of 80°C to 180°C and a peak at 140.3 °C.

All the batches were tested for ammonia residue by comparing with 1 ppm ammonia standard; ammonia was below detection limit in all three batches.

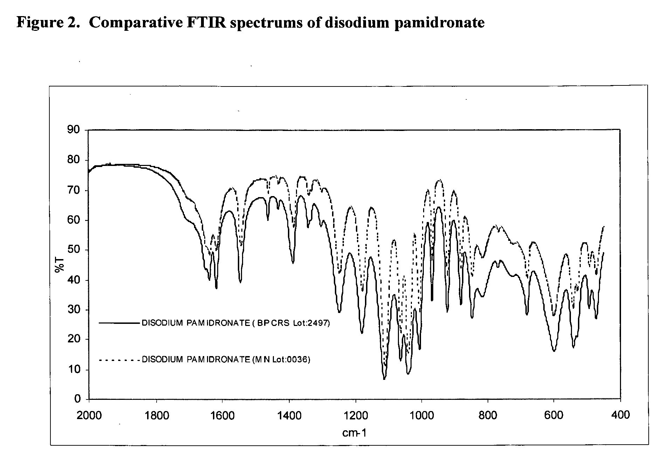

All the batches gave excellent conformity with the FTIR spectrum of disodium pamidronate British Pharmacopoeia CRS lot: 2497 (see Figure 2).

Assay values were found to be within the range of 100.2 to 100.6 % (ODB) against disodium pamidronate BP CRS lot: 2497.

GC Head-space residual solvent analysis showed that there was no residual solvent detectable in any chromatogram for the three batches produced.

Stability of the product is evident from the fact that there was no loss of water, when the product was stored at room temperature at reduced pressure (20 millibar), as we found by KF method 24.7 % water after 4 hours of storage, 24.4 % water after 10 hours of storage, 24.35 %, 24.38 %, 24.32 % of water after 38 hours of storage (the 38 h values were determined in triplicate).

These investigations were carried out in a HERAEUS vacuum drying oven of a volume of 53 liters, at a relative humidity (RH) of less than 1 %.
Investigations of storage stability at normal pressure were carried out over a period of 1 week, in compliance with the internationally accepted standards about storage stability test conditions for pharmaceuticals as defined in ICH (International Conference on Harmonization of Technical Requirements for Registration of Pharmaceuticals for Human Use), at 25°C / 60 % RH for longterm stability condition, 30°C / 60 % RH for intermediate term stability condition and 40°C / 75 % RH for accelerated stability condition.

The results (see Table 1) clearly show that the pentahydrate of disodium pamidronate as produced by the method of the presented invention is stable under the above conditions.

Moreover, according to these data, we wonder what kind of pentahydrate KIM & al. investigated for stability, when they stated in experimental example 1 of their US patent 6,534,674: "However, in the case of 5 hydrates, a part of the water contents (24,41 %) is lost from 1 week under the conditions of 24°C, 50 % RH and the 5 hydrates becomes to 3-4 hydrates" (column 10, lines 2-5).

**Table 1. Water content (%, KF) of 3 lots of disodium pamidronate after storage for 1 week**

| Lot no. | initial | 25°C/60%RH | 30°C/60%RH | 40°C/75%RH |
|---|---|---|---|---|
| 0034 | 24.55 | 24.37 | 24.38 | 24.43 |
| 0035 | 24.38 | 24.40 | 24.39 | 24.27 |
| 0036 | 24.48 | 24.32 | 24.35 | 24.39 |

### Example 1: Neutralization of pamidronic acid with ammonia

60 grams of pamidronic acid was dispersed into 80 ml of water in the jacketed calcium free glass titration vessel while stirring with Teflon^{®} coated magnetic stirrer. This pamidronic acid was titrated at room temperature with ammonia solution of a concentration of 12.5 % (w/v) up to second inflection point, namely pH 7.55. The temperature of the titrated solution went up to 35°C (due to the exothermic reaction) and the temperature was lowered via cooling jacket from 35°C to 30°C. Then this diammonium pamidronate solution was passed through hydrophilic polypropylene membrane filter of 0.45 micron and transferred to another calcium free jacketed glass vessel.

### Example 2: Ion-exchanging and purification

The titrated and filtered diammonium pamidronate solution was passed via peristaltic pump through a column filled with AMBERLITE^{®} SR1L Na (strongly acidic cation exchange resin, food grade), which had been pre-purified and regenerated in reverse flow direction according to producer's recommendation with hydrochloric acid solution at a concentration of 7 % (w/v) and sodium hydroxide solution at a concentration of 16 % (w/v) at three consecutive alternate cycles to remove some ppm levels of monomers and organic compounds. Then the system was flushed with water for injection having a TOC level of 80 ppb. Sample were taken from the outlet tubing of the column and analyzed for TOC, which was found to be 95 ppb at 50°C. By the above procedure all divalent and trivalent ions of metals such as calcium, magnesium, iron, chromium and aluminium, known to form undesired complexes with pamidronate are removed.
The resin bed volume was selected in a way that at least it had a capacity of 1.5 times the equivalent of the passed solution (in our application it was 1.7). The column was equilibrated at 50°C for half an hour before the filtered diammonium pamidronate solution as obtained from the titration step was passed through it.

### Example 3: Crystallization of Disodium pamidronate

The purified disodium pamidronate solution (190 ml) from the ion-exchange column was collected in a calcium free jacketed crystallization vessel equilibrated at 50°C while stirring with Teflon® coated magnetic stirrer. The temperature of the crystallization vessel was cooled down from 50°C to 5°C in one hour. Crystallization was starting at a temperature of 45°C spontaneously. After maintaining the temperature at 5°C for half an hour, a thick slurry of disodium pamidronate pentahydrate crystals was obtained and transferred to water-jet equipped vacuum filtration system. Separated disodium pamidronate pentahydrate crystals were dried in the vacuum oven at the pressure of 10 millibar for 4 hours at room temperature. After drying, pure disodium pamidronate pentahydrate crystals were obtained and weighed as 88.1 grams; this corresponded to a yield of 94 %. Water content was determined by KF to be 24.48 %. The above procedure was carried out in total for three times. The results are given in Table 2 with the analytical data obtained for water, assay and yield.

**Table 2. Analysis results of disodium pamidronate pentahydrate produced with the above procedure.**

| Lot no. | Water % (KF) | Assay %(ODB) | Yield % |
|---|---|---|---|
| 0034 | 24.55 | 100.2 | 93.5 |
| 0035 | 24.38 | 100.6 | 94.2 |
| 0036 | 24.48 | 100.4 | 93.9 |
| Average | 24.47 | 100.4 | 93.9 |

### X-Ray Powder Diffraction Pattern

The obtained disodium pamidronate pentahydrate crystals are characterized by the following lattice spacings (d-values) and line intensities of its X-ray powder diffraction pattern. Data were obtained with Shimadzu XRD-6000 (Radiation source: copper-Kα).

| **d-value (Angstrom)** | **Intensity** | **Evaluation** |
|---|---|---|
| 10.85 | 22 | very weak |
| 10.22 | 223 | medium |
| 9.88 | 231 | strong |
| 9.23 | 408 | very strong |
| 5.92 | 463 | very strong |
| 5.58 | 327 | very strong |
| 5.41 | 60 | very weak |
| 5.31 | 125 | weak |
| 5.14 | 181 | weak |
| 4.98 | 367 | very strong |
| 4.41 | 57 | very weak |
| 4.16 | 103 | weak |
| 4.04 | 91 | weak |
| 3.75 | 208 | medium |
| 3.67 | 25 | very weak |
| 3.61 | 89 | weak |
| 3.51 | 145 | weak |
| 3.43 | 227 | medium |
| 3.37 | 27 | very weak |
| 3.14 | 83 | very weak |
| 3.08 | 71 | very weak |
| 3.02 | 649 | very strong |
| 2.97 | 165 | medium |
| 2.91 | 178 | medium |
| 2.82 | 159 | medium |
| 2.78 | 161 | weak |
| 2.75 | 62 | very weak |
| 2.71 | 109 | very weak |
| 2.67 | 50 | very weak |
| 2.63 | 146 | medium |
| 2.60 | 143 | medium |
| 2.57 | 524 | very strong |
| 2.48 | 72 | very weak |
| 2.45 | 74 | very weak |
| 2.40 | 20 | very weak |
| 2.35 | 89 | medium |
| 2.32 | 44 | very weak |
| 2.30 | 41 | very weak |
| 2.26 | 109 | medium |
| 2.17 | 56 | very weak |

## Claims

1. A process for manufacturing disodium salt of pamidronic acid **characterized in**
- **that** the acid is titrated with ammonia unto a pH of 7.50-7.60,
- the resulting solution is passed over the sodium form of an ion-exchange column at elevated temperature,
- the resulting eluate containing disodium pamidronate is cooled down to lower temperature until the disodium salt of pamidronic acid is crystallizing,
- and the crystals are harvested by centrifugation or vacuum filtration and dried in vacuum at 25°C.

2. A process according to claim no.1 **characterized in**
**that** the concentration of ammonia used is from 3 % to 30 %, preferably 12.5 % (w/v).

3. A process according to claim no.1 **characterized in**
**that** the total amount of the ammonia used is such that the final pH of the neutralized solution is 7.55 ± 0.05 at 30°C.

4. A process according to claim no.1 **characterized in**
**that** the temperature at which the treatment of the acid with the ammonia is carried out is in a temperature range between 20°C to 40°C, preferably at 30°C.

5. A process according to claim no.1 **characterized in**
**that** the diammonium pamidronate solution is passed over the ion-exchange column at a temperature of 40°C to 60°C, preferably 50°C.

6. A process according to claim no.5 **characterized in**
**that** ion-exchange resin is a strongly acidic cation exchange resin in its sodium form.

7. A process according to claim no.6 **characterized in**
**that** the eluate containing disodium pamidronate is being crystallized by lowering the temperature to 5°C.

8. A process according to claim no.7 **characterized in**
**that** the crystals of disodium pamidronate are harvested and dried at room temperature under the vacuum at 2 to 20 millibar.

## Patentansprüche

1. Verfahren zur Herstellung von Dinatriumsalz von Pamidronsäure, **dadurch gekennzeichnet, dass**
- die Säure mit Ammoniak bis zu einem pH-Wert von 7,50-7,60 titriert wird,
- die erhaltene Lösung bei erhöhter Temperatur über die Natriumform einer Ionenaustauschersäule geführt wird,
- das erhaltene Eluat, das Dinatriumpamidronat enthält, auf eine niedrigere Temperatur abgekühlt wird, bis das Dinatriumsalz von Pamidronsäure kristallisiert,
- und die Kristalle durch Zentrifugation oder Vakuumfiltration gesammelt und bei 25°C im Vakuum getrocknet werden.

2. Verfahren nach Anspruch 1, **dadurch gekennzeichnet, dass** die verwendete Ammoniakkonzentration 3 bis 30% und vorzugsweise 12,5% (w/v) beträgt.

3. Verfahren nach Anspruch 1, **dadurch gekennzeichnet, dass** die Gesamtmenge des verwendeten Ammoniaks so beschaffen ist, dass der End-pH-Wert der neutralisierten Lösung bei 30°C 7,55 ± 0,05 beträgt.

4. Verfahren nach Anspruch 1, **dadurch gekennzeichnet, dass** die Temperatur, bei der die Behandlung der Säure mit dem Ammoniak durchgeführt wird, in einem Temperaturbereich zwischen 20°C und 40°C, vorzugsweise bei 30°C, liegt.

5. Verfahren nach Anspruch 1, **dadurch gekennzeichnet, dass** die Diammoniumopamidronat-Lösung bei einer Temperatur von 40°C bis 60°C, vorzugsweise 50°C, über die Ionenaustauschersäule geführt wird.

6. Verfahren nach Anspruch 5, **dadurch gekennzeichnet, dass** es sich bei dem Ionenaustauscherharz um ein stark saures Kationenaustauscherharz in seiner Natriumform handelt.

7. Verfahren nach Anspruch 6, **dadurch gekennzeichnet, dass** das Eluat, das Dinatriumpamidronat enthält, durch Senkung der Temperatur auf 5°C kristallisiert wird.

8. Verfahren nach Anspruch 7, **dadurch gekennzeichnet, dass** die Kristalle von Dinatriumpamidronat gesammelt und bei Raumtemperatur unter einem Vakuum von 2 bis 20 Millibar getrocknet werden.

## Revendications

1. Procédé de fabrication de sel disodique d'acide pamidronique **caractérisé en ce que**
- l'acide est titré avec de l'ammoniac jusqu'à un pH de 7,50 à 7,60,
- la solution résultante est passée sur la forme sodique d'une colonne échangeuse d'ions à une température élevée,
- l'éluat résultant contenant du pamidronate disodique est refroidi jusqu'à une température inférieure jusqu'à ce que le sel disodique d'acide pamidronique cristallise, et
- les cristaux sont recueillis par centrifugation ou filtration sous vide et séchés sous vide à 25 °C.

2. Procédé selon la revendication 1, **caractérisé en ce que** la concentration d'ammoniac utilisée est comprise entre 3 % et 30 %, de préférence est de 12,5 % (p/v).

3. Procédé selon la revendication 1, **caractérisé en ce que** la quantité totale d'ammoniac utilisée est telle que le pH final de la solution neutralisée est de 7,55 ± 0,05 à 30 °C.

4. Procédé selon la revendication 1, **caractérisé en ce que** la température à laquelle est réalisé le traitement de l'acide avec l'ammoniac est située dans la plage de températures allant de 20 °C à 40 °C, de préférence est de 30 °C.

5. Procédé selon la revendication 1, **caractérisé en ce que** la solution de pamidronate de diammonium est passée sur la colonne échangeuse d'ions à une température de 40 °C à 60 °C, de préférence à 50 °C.

6. Procédé selon la revendication 5, **caractérisé en ce que** la résine échangeuse d'ions est une résine échangeuse de cations fortement acide sous sa forme sodique.

7. Procédé selon la revendication 6, **caractérisé en ce que** l'éluat contenant du pamidronate disodique est cristallisé en abaissant la température à 5 °C.

8. Procédé selon la revendication 7, **caractérisé en ce que** les cristaux de pamidronate disodique sont recueillis et séchés à température ambiante sous vide à 2 à 20 millibars.
